# FASCICULE DE BREVET EUROPEEN

(11) **EP 2 239 272 B1**
(45) Date de publication et mention de la délivrance du brevet: **01.01.2014**
(21) Numéro de dépôt: 10007471.5
(22) Date de dépôt: 18.11.2003
(51) Int. Cl.: C07K 16/28, G01N 33/53, C07K 14/705, C12N 15/12, C12Q 1/68

(54) **Protéine spécifique des cellules pancréatiques bêta des îlots de Langerhans et ses applications**
Spezielles Eiweiß der pankreatischen Beta-Zellen der Langerhans-Inseln und seine Anwendungen
Specific protein of the pancreatic beta cells of the Langerhans' islets and its applications

(30) Priorité: 18.11.2002 FR 0214374
(43) Date de publication de la demande: 13.10.2010
(62) Demande divisionnaire de: 08002520.8
(73) Titulaire: Commissariat à l'Énergie Atomique et aux Énergies Alternatives, 75015 Paris (FR); Université Joseph Fourier, 38041 Grenoble Cédex 9 (FR)
(72) Inventeur: Seve, Michel, 38240 Meylan (FR); Favier, Alain, 38190 Bernin (FR)
(74) Mandataire: Marcadé, Véronique

(56) Documents cités:
- WO-A-01/42789
- WO-A-01/94409
- WO-A-02/24733
- WO-A2-02/060953
- DATABASE EMBL [Online] ik02a02.y1 Human insulinoma 9 mai 2002 (2002-05-09), MELTON D. ET AL.: "Homo sapiens cDNA clone IMAGE: 5779491 5' similar to TR:Q62941 zinc transporter ZNT-2; mRNA sequence" XP002246606 extrait de EBI Database accession no. BQ267316
- DATABASE EMBL [Online] ik36b01.y1 HR85 islet MELTON D. ET AL.: "Homo sapiens cDNA clone IMAGE: 5782969 5' similar to TR:Q62941 zinc transporter ZNT-2; mRNA sequence" XP002246607 extrait de EBI Database accession no. BQ417284
- DATABASE EMBL [Online] in61h03.y1 HR85 islet MELTON D. ET AL.: "Homo sapiens cDNA clone IMAGE: 5782969 5' similar to TR:Q62941 Zinc transporter ZNT-2; mRNA sequence" XP002246608 extrait de EBI Database accession no. BU949895
- TOMITA T: "New markers for pancreatic islets and islet cell tumors" PATHOLOGY INTERNATIONAL 2002 JAPAN, vol. 52, no. 7, 2002, pages 425-432, XP002246605 ISSN: 1320-5463
- KROGH A ET AL: "Predicting transmembrane protein topology with a hidden markov model: application to complete genomes" JOURNAL OF MOLECULAR BIOLOGY, LONDON, GB LNKD- DOI:10.1006/JMBI.2000.4315, vol. 305, no. 3, 19 janvier 2001 (2001-01-19), pages 567-580, XP004469188 ISSN: 0022-2836

## Description

La présente Invention est relative à une protéine dénommée ZnT-8, exprimée de manière spécifique dans les cellules pancréatiques bêta des îlots de Langerhans, au polynucléotide codant ladite protéine qui est impliquée dans la maturation et l'exocytose de l'insuline, ainsi qu'à leurs applications notamment pour le tri et l'étude des cellules bêta, ainsi que pour le criblage de médicaments actifs sur le diabète et l'hyperinsulinisme.

Le diabète est une des maladies les plus fréquentes, affectant 5 % de la population des pays industrialisés et en constante augmentation dans tous les pays du monde (prévision : 300 millions en 2025, dont 2,4 millions en France). Parmi les diverses formes de diabète, le diabète de type I ou insulino-dépendant affecte environ 500 000 à 1 million d'individus aux Etats-Unis et 150 000 en France soit 0,2 à 0,4 % de la population. Les symptômes caractéristiques comprennent un taux élevé de sucre dans le sang et dans les urines, une diurèse importante, une faim et une soif intenses ainsi qu'une perte de poids.

Le diabète de type II non insulino-dépendant (DNID), aussi décrit sous le nom de diabète "gras" ou diabète de la maturité, survient souvent autour de la cinquantaine. Il est traité par un régime, la prise de médicaments par voie orale, et l'insuline, après quelques années d'évolution. Aujourd'hui, 2 millions de Français sont traités avec des médicaments antidiabétiques et/ou de l'insuline.

Bien que le diabète puisse être contrôlé par des injections d'insuline et des apports contrôlés en glucides, les complications associées à cette pathologie nécessitent de nos jours de nouvelles approches, dans sa prévention, son traitement et son diagnostic.

Le pancréas comprend deux structures distinctes aussi bien morphologiquement que physiologiquement :
- le pancréas exocrine qui produit les enzymes intervenant dans la digestion (amylase, lipase, etc.) et le bicarbonate de sodium ;
- le pancréas endocrine qui produit les hormones intervenant dans le contrôle du glucose sanguin (insuline, glucagon, somatostatine et polypeptide pancréatique).

Les cellules du pancréas endocrine sont organisées en micro-organes dispersés dans le pancréas sous forme d'îlots (îlots de Langerhans ou îlots pancréatiques). Chaque îlot pancréatique est composé de 4 types cellulaires : les cellules alpha, bêta, delta, et les cellules PP. Les cellules alpha sont situées à la périphérie de l'îlot et secrètent le glucagon. Les cellules bêta sont retrouvées au centre de l'îlot et sont les seules cellules capables de secréter l'insuline en réponse au glucose. Les cellules delta sont à la périphérie et secrètent la somatostatine. La fonction des cellules PP est plus controversée (synthèse du polypeptide pancréatique).

L'absence de modèle cellulaire d'étude de la cellule bêta, ainsi que l'absence de moyen fiable et efficace de tri cellulaire adapté à ce type de cellule, freine l'étude de son fonctionnement et donc la mise au point de nouvelles méthodes de traitement du diabète de type I et II.

Parmi les traitements du diabète, outre l'administration régulière d'insuline, une des voies du contrôle physiologique de la glycémie et de la normalisation de la glycémie chez les diabétiques est le passage par la restauration d'une sécrétion d'insuline *in vivo* à partir de cellules. Dans cette optique plusieurs solutions ont été proposées :
- l'obtention de cellules productrices d'insuline chez l'animal pour la réalisation d'une xénotransplantation ;
- la différenciation *in vitro* en cellules sécrétrices d'insuline à partir de cellules souches isolées, dans un but de réimplantation [1], ceci afin de contourner les problèmes d'immunité et la nécessité d'un traitement immunosuppresseur du patient. Mais la production de quantités importantes de cellules à faible coût, productrices d'insuline par différenciation de cellules souches, nécessite de nouveaux outils biomoléculaires, notamment utiles pour le phénotypage et la purification des cellules différenciées ;
- la transplantation d'îlots pancréatiques ; de nombreux travaux ont eu récemment pour objet la préparation d'îlots ou de cellules bêta pancréatiques à des fins thérapeutiques. La première étape de la transplantation est le prélèvement du pancréas sur un donneur en état de mort cérébrale. L'isolement des îlots commence par une digestion enzymatique du pancréas au moyen d'une solution de collagénase. Toutes les transplantations ne requièrent pas une purification des îlots digérés. Toutefois, la plupart des chercheurs s'accordent aujourd'hui pour dire que la purification des îlots est nécessaire pour une allotransplantation [2]. Les îlots sont ensuite transplantés, à raison d'une masse suffisante (minimum 3000 IEQ/kg) par injection intraportale (IEQ : Islet Equivalent, Equivalent Ilôt).

Toutefois, l'isolement d'îlots ou de cellules bêta pancréatiques nécessite des moyens de sélection et d'identification des cellules bêta, spécifiques et fiables.

Des travaux antérieurs ont tenté de mettre au point des méthodes de marquage des cellules bêta. On peut citer :
- le marquage par la GFP (Green Fluorescent Protein) qui permet un marquage fluorescent de la cellule. L'inconvénient majeur de cette technique est la nécessité d'introduire un gène exogène ou transgène dans la cellule, qui plus est par l'utilisation d'un vecteur viral (adénovirus) [1] ;
- la technique basée sur l'autofluorescence importante de la cellule bêta [2]. Mais cette technique manque de spécificité vis-à-vis du type cellulaire ;
- l'incubation des cellules avec un fluorochrome spécifique du zinc : le Newport Green [3] ou la dithizone [4]. Cette technique est basée sur la teneur importante en zinc de la cellule bêta. Le zinc est un constituant important des granules de sécrétion de l'insuline, et en outre, joue un rôle dans le contrôle de cette sécrétion [5]. Mais ces techniques présentent de nombreux inconvénients : utilisation d'un produit chimique, risque de toxicité vis-à-vis de la cellule bêta, manque de spécificité vis-à-vis du type cellulaire. De plus, la dithizone pose un problème de photodégradation rapide [6] ;
- la mise en évidence de manière indirecte par reconnaissance par un clone de cellules T (WO 91/17186) d'un antigène exprimé par les cellules bêta. Les travaux initiaux sur cet antigène n'apportaient pas de résultats en terme de caractérisation de la séquence peptidique, ainsi qu'en terme de sélectivité de la cellule bêta par rapport aux autres types cellulaires de l'îlot, mais également du pancréas ou de l'organisme. Des travaux plus récents des mêmes auteurs montrent une distribution beaucoup plus large de cet antigène, qui de fait n'est pas spécifique de la cellule bêta [7] ;
- la détection du transporteur du glucose GLUT-2 [15] ; mais ce transporteur n'est pas spécifique des cellules bêta.

En conséquence, il existe un manque de marqueur spécifique et fiable de la cellule bêta des îlots pancréatiques de Langerhans.

C'est un des buts de la présente étude que de fournir un tel marqueur.

L'îlot de Langerhans accumule de très grandes quantités de zinc et nécessite donc un transporteur très efficace et très spécialisé pour accumuler ce zinc dans les vésicules de sécrétion [8]. L'insuline, produite et stockée dans la cellule pancréatique bêta, est libérée dans le milieu extracellulaire par exocytose en réponse à des stimuli externes, comme une élévation de la concentration en glucose. Cette élévation du glucose provoque une modification du rapport ATP/ADP, la fermeture des canaux potassiques et l'ouverture des canaux calciques qui provoque l'exocytose [9].

Il est connu qu'en présence de zinc, l'insuline peut former des tétramères et des hexamères qui fixent le zinc dans un rapport insuline : zinc de 4 :1 et 6 :2 respectivement. L'insuline est stockée dans les granules de sécrétion sous la forme d'un solide composé d'hexamères liés à 2 atomes de zinc par hexamère. Les vésicules contiennent du zinc en un excès de 1 à 1,5 fois la quantité nécessaire pour former les hexamères insuline-zinc. Au cours de l'exocytose de l'insuline, les vésicules riches en insuline fusionnent avec la membrane plasmique de la cellule bêta et libèrent l'insuline mais également le zinc, dans la circulation. Le zinc libéré agit dans une boucle de rétrocontrôle négatif sur les canaux potassiques, provoquant son activation et l'arrêt de l'exocytose.

Dans les cellules de mammifères, 7 protéines homologues ayant une fonction de transporteur du zinc, dénommées ZnT-1, -2, -3, -4, -5, -6 et -7, ont été clonées et caractérisées. L'analyse de la structure primaire de ces protéines a permis de définir un motif structural commun composé de 6 domaines transmembranaires et d'une boucle intracellulaire riche en histidine. ZnT-1 est un transporteur ubiquitaire localisé dans la membrane plasmique qui assure l'efflux de zinc hors de la cellule [11]. ZnT-2 permet à la cellule de tolérer un excès de zinc dans le milieu de culture, conférant ainsi une résistance au zinc par une localisation de celui-ci dans des vésicules intracytoplasmiques acides, assurant ainsi une accumulation du zinc dans la cellule bien supérieure à la normale [12]. ZnT-3 et ZnT-4 clonés chez l'homme, ont des fonctions similaires à ZnT-2. ZnT-3 est spécifique de certains tissus et fortement exprimée dans le cerveau, dans les membranes des vésicules synaptiques riches en zinc, dans les fibres moussues de l'hippocampe et dans les testicules. ZnT-4 est exprimée de manière ubiquitaire, mais des niveaux plus élevés sont retrouvés dans le cerveau et les cellules épithéliales. Ce transporteur est essentiel dans l'épithélium mammaire où il participe au contrôle du contenu en zinc du lait maternel. ZnT-5 et ZnT-6 sont aussi des transporteurs ubiquitaires localisés dans l'appareil de Golgi. ZnT-7 est un transporteur ubiquitaire, localisé spécifiquement dans le réticulum endoplasmique des cellules.

Un polynucléotide codant un polypeptide transporteur de Zinc appelé NOV2 a également été décrit dans la Demande Internationale WO 02/24733. Ledit polypeptide est exprimé au niveau non seulement du pancréas mais aussi de la moelle osseuse, du cartilage, du placenta et du rein. Par conséquent, du fait de son expression dans plusieurs tissus, ce polypeptide ne peut être utilisé en tant que marqueur spécifique des cellules bêta-pancréatiques.

Des travaux précédents mentionnent une tentative de recherche de gènes impliqués dans le métabolisme du zinc dans la cellule bêta pancréatique. Ces travaux n'ont pas abouti à la mise en évidence d'une protéine ou d'un transporteur spécifique [10].

Les Inventeurs ont isolé un polynucléotide de 1110 paires de bases (SEQ ID NO:1) représentant l'ADNc correspondant à un ARNm exprimé de manière spécifique dans l'îlot de Langerhans, et plus particulièrement dans la cellule sécrétrice d'insuline ou cellule bêta.

Ce polynucléotide code pour une protéine dénommée ZnT-8, (SEQ ID NO:2), laquelle protéine de 369 acides aminés correspondant à une masse moléculaire estimée à 40,8 KDa, présente une structure primaire homologue à celle des protéines de la famille ZnT. Le gène codant ladite protéine est dénommé *ZnT-8.*

L'utilisation d'un polypeptide comprenant la séquence SEQ ID NO : 2 pour la détection, chez un sujet, d'anticorps dirigés contre ce polypeptide a été décrite, dans le cadre d'une méthode de diagnostic du cancer du pancréas, dans la Demande Internationale WO 01/94409. Cependant, un tel polypeptide n'y est pas décrit comme étant spécifique de la cellule bêta des îlots de Langerhans.

L'étude du potentiel transmembranaire de la protéine ZnT-8 complète montre qu'elle possède 6 domaines transmembranaires (acides aminés 74-95, 107-126, 141-163, 177-196, 216-240, 246-267), les extrémités N- et C-terminales étant situées dans le cytoplasme. Cette étude montre également que la structure secondaire de cette protéine présente 3 boucles d'acides aminés extracellulaires (acides aminés 96-106, 164-176, 241-245).

En outre, la localisation de la protéine ZnT-8 dans les vésicules de sécrétion de l'Insuline et sur la membrane plasmique indique qu'elle est impliquée dans l'accumulation du zinc dans les vésicules contenant l'insuline et joue donc un rôle dans la maturation et l'exocytose de l'insuline dans les cellules bêta des îlots pancréatiques de Langherans.

La protéine ZnT-8 et le polynucléotide correspondant constituent pour la première fois un marqueur spécifique et fiable de la cellule bêta des îlots pancréatiques de Langerhans ; les utilisations de ce marqueur sont notamment les suivantes :
- tri cellulaire ; le marqueur permet d'envisager un tri et un repérage sélectif des cellules bêta, sans modification chimique ou biologique desdites cellules, notamment à l'aide d'anticorps dirigés contre ladite protéine.
- modèle d'étude *in vitro* ; le marqueur peut être utilisé *in vitro* pour étudier : (i) la surexpression du transporteur (ZnT-8) dans des lignées de cellules modèles (insulinome de rat INS-1 par exemple) et de l'impact sur la sécrétion de l'insuline en réponse à une stimulation par le glucose, (ii) la sensibilité des cellules à la mort cellulaire (apoptose) induite par des conditions de stress oxydant ou de concentration faible ou forte en zinc, et (iii) les étapes de différenciation de cellules souches en cellules sécrétrices d'insuline en réponse à différentes stimulations exogène (facteurs de croissance, extraits pancréatiques).
- criblage de médicaments : le marqueur représente également une cible pharmacologique utile pour le criblage de substances capables de moduler l'expression du gène *ZnT-8* et/ou l'activité de la protéine ZnT-8, potentiellement utilisables pour le traitement du diabète et de l'hyperinsulinisme.
- diagnostic du diabète : le polynucléotide peut également avantageusement être mis en oeuvre dans le diagnostic précoce du diabète dans les familles à risque, notamment en ce qu'il permet de détecter des mutations observables dans le gène *ZnT-8* et aussi de diminuer voir de supprimer les examens habituellement mis en oeuvre.

Ainsi, les Inventeurs décrivent l'utilisation en tant que marqueur spécifique des cellules bêta des îlots pancréatiques de Langherans d'au moins un polynucléotide isolé ou de la protéine correspondante choisis parmi :
- les polynucléotides comprenant ou présentant l'une des séquences suivantes : (a) la séquence SEQ ID NO:1, (b) un fragment de la séquence SEQ ID NO:1 d'au moins 15 nucléotides consécutifs, de préférence 20 nucléotides, encore plus préférentiellement 25 à 30 nucléotides, (c) une séquence présentant un pourcentage d'identité d'au moins 80 % après alignement optimal avec l'une des séquences définies en (a) ou en (b), et (d) une séquence complémentaire, sens ou antisens de l'une des séquences définies en (a), (b) ou (c), et
- les protéines codées par les polynucléotides tels que définis en (a), (b), (c) ou (d) ci-dessus comprenant ou présentant l'une des séquences suivantes : (e) la séquence SEQ ID NO:2, (f) un fragment de la séquence SEQ ID NO:2 d'au moins 15 acides aminés consécutifs, (g) une séquence présentant un pourcentage d'identité d'au moins 60 % après alignement optimal avec l'une des séquences définies en (e) ou en (f) ou au moins 65 % de similarité, de préférence 80 % d'identité ou au moins 90 % de similarité, ou de manière encore plus préférée 90 % d'identité ou au moins 95 % de similarité.

Le polynucléotide tel que défini ci-dessus, peut être isolé à partir de cellules de Langerhans ou à partir de banques d'ADN cellulaire, particulièrement de banques d'ADN de cellules pancréatiques, très particulièrement à partir d'une banque d'ADN de cellules pancréatiques humaines. De préférence, les cellules utilisées sont des cellules des îlots de Langerhans.

Le polynucléotide tel que défini ci-dessus peut également être obtenu par une réaction de polymérisation en chaîne (PCR) effectuée sur l'ADN total des cellules de Langerhans, par RT-PCR effectuée sur les ARN totaux des cellules bêta d'îlots pancréatiques de Langerhans ou par synthèse chimique.

Par polynucléotide, on entend un enchaînement précis de nucléotides, modifiés ou non, comportant ou non des nucléotides non naturels. Ainsi, ce terme recouvre toute séquence qui code pour une protéine ZnT-8 ou un fragment de ladite protéine (ADN génomique, ARNm, ADNc) mais aussi les oligonucléotides, sens ou antisens, ainsi que les petits ARN interférents (small interfering RNA, siRNA) correspondants.

Par "acides nucléiques ou protéines présentant un pourcentage d'identité après alignement optimal avec une séquence de référence", on entend désigner les acides nucléiques ou les protéines présentant, par rapport à la séquence de référence, certaines modifications comme en particulier une délétion, une troncation, un allongement, une fusion chimérique, et/ou une substitution, notamment ponctuelle, et dont la séquence en nucléotides présente au moins 80 % d'identité et la séquence en acides aminés présente au moins 65 % d'identité après alignement optimal avec la séquence en nucléotides ou en acides aminés de référence.

Par "pourcentage d'identité" entre deux séquences (acides nucléiques ou protéines), on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur.

Par "meilleur alignement" ou "alignement optimal", on entend désigner l'alignement pour lequel le pourcentage d'identité déterminé comme décrit ci-après est le plus élevé. Les comparaisons entre deux séquences de nucléotides ou d'acides aminés sont traditionnellement réalisées : en comparant ces séquences après les avoir alignées de manière optimale, ladite comparaison étant réalisée par segment ou par "fenêtre de comparaison" pour identifier et comparer les régions locales de similarité de séquence. L'alignement optimal des séquences pour la comparaison peut être réalisé, notamment à l'aide de l'un des algorithmes suivants : l'algorithme d'homologie locale de Smith et Waterman (1981), l'algorithme d'homologie locale de Neddleman et Wunsch (1970), la méthode de recherche de similarité de Pearson et Lipman (1988), les logiciels informatiques utilisant ces algorithmes (GAP, BESTFIT, BLAST P, BLAST N, BLASTX, TBLASTX, FASTA et TFASTA dans le Wisconsin Genetics Software Package, (Genetics Computer Group, 575 Science Dr., Madison, WI) ou sur les serveurs internet, en particulier ceux du *National Center for Biotechnology Information* (http ://www.ncbi.nlm.nih.gov), de l'EMBL (http ://www.embl.org) et du projet Ensembl (http ://www.ensembl.org)).

Afin d'obtenir l'alignement optimal, on utilise de préférence le programme BLAST, avec la matrice BLOSUM 62. On peut également utiliser les matrices PAM ou PAM250, ainsi qu'une matrice d'identité pour les séquences de nucléotides.

Pour obtenir une "hybridation spécifique" on utilise de préférence des conditions d'hybridation de forte stringence, c'est-à-dire des conditions de température et de force ionique choisies de telle manière qu'elles permettent le maintien de l'hybridation spécifique et sélective entre polynucléotides complémentaires.

A titre d'illustration, des conditions de forte stringence de l'étape d'hybridation aux fins de définir les polynucléotides décrits ci-dessus, sont avantageusement les suivantes : l'hybridation ADN-ADN ou ADN-ARN est réalisée en deux étapes : (1) préhybridation à 42°C pendant 3 heures en tampon phosphate (20 mM pH 7,5) contenant 5 x SSC (1 x SSC correspond à une solution 0,15 M NaCl + 0, 015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille supérieure à 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille supérieure à 100 nucléotides. Les conditions d'hybridation de forte stringence décrites ci-dessus pour une sonde de taille définie peuvent être adaptées par l'Homme du Métier pour des sondes de taille plus grande ou plus petite.

Par "techniques ou méthodes appropriées" on entend ici se référer aux techniques ou méthodes bien connues et classiquement utilisées par l'Homme du Métier et exposées dans de nombreux ouvrages, comme en particulier celui intitulé Molecular Cloning. A Laboratory Manual (Sambrook J, Russell DW. (2000) Cold Spring Harbor Laboratory Press) [13].

Le polynucléotide tel que défini en c) présente un pourcentage d'identité d'au moins 80 % après alignement optimal avec une séquence telle que définie en a) ou b), de préférence 90 %, de façon plus préférée 95 %, de façon encore plus préférée 98 %. Le polynucléotide tel que défini en c) inclut les polynucléotides variants de la séquence SEQ ID NO:1, c'est-à-dire l'ensemble des polynucléotides correspondants à des variants alléliques, c'est-à-dire à des variations individuelles de la séquence SEQ ID NO:1. Ces séquences variantes naturelles correspondent à des polymorphismes présents chez les mammifères, en particulier chez l'être humain et, notamment à des polymorphismes pouvant conduire à la survenue d'une pathologie, comme par exemple la mort cellulaire des îlots de Langerhans et un diabète.

On entend également désigner par polynucléotide variant, tout ARN ou ADNc résultant d'une mutation et/ou d'une variation d'un site d'épissage de la séquence génomique dont l'ARNm a comme ADN complémentaire le polynucléotide de séquence SEQ ID NO:1.

La similarité d'une protéine par rapport à une protéine de référence s'apprécie en fonction du pourcentage de résidus d'acides aminés qui sont identiques ou qui différent par des substitutions conservatives, lorsque les deux séquences sont alignées de manière à obtenir le maximum de correspondance entre elles. On entend par substitution conservative, la substitution d'un acide aminé par un autre qui présente des propriétés chimiques similaires (taille, charge ou polarité), qui généralement ne modifie pas les propriétés fonctionnelles de la protéine.

Une protéine ayant une séquence en acides aminés ayant au moins X % de similarité avec une séquence de référence est définie comme une protéine dont la séquence peut inclure jusqu'à 100-X altérations non-conservatives pour 100 acides aminés de la séquence de référence. Le terme altérations non-conservatives inclut les délétions, les substitutions non-conservatives ou les insertions consécutives ou dispersées d'acides aminés dans la séquence de référence.

Sont incluses dans les protéines telles que définies en (g) les protéines variantes de la séquence SEQ ID NO:2, c'est-à-dire les protéines variantes codées par les polynucléotides variants tels que précédemment définis, en particulier les protéines dont la séquence en acides aminés présente au moins une mutation correspondant notamment à une troncation, une délétion, une substitution et/ou une addition d'au moins un résidu d'acide aminé par rapport à la séquence SEQ ID NO:2.

De manière préférée, les protéines variantes présentent une mutation associée au diabète ou à l'hyperinsulinisme.

Selon un mode de réalisation avantageux de l'utilisation telle que définie ci-dessus, ledit polynucléotide isolé tel que défini en (c), est un polynucléotide variant de la séquence SEQ ID NO:1 comportant une mutation qui conduit à une modification de la séquence en acides aminés de la protéine codée par la séquence SEQ ID NO:1.

Selon un autre mode de réalisation avantageux de l'utilisation telle que définie ci-dessus, lesdits polynucléotides isolés tel que définis en (b) ou en (d) sont choisis parmi la paires d'amorces SEQ ID NO:3 et SEQ ID NO:4 et la paire d'amorce SEQ ID NO:5 et SEQ ID NO:6.

Selon encore un autre mode de réalisation avantageux de l'utilisation telle que définie ci-dessus, ledit polynucléotide isolé est susceptible d'être obtenu par amplification à l'aide de la paire d'amorces telle que définie ci-dessus.

Selon encore un autre mode de réalisation avantageux de l'utilisation telle que définie ci-dessus, ledit polynucléotide tel que défini en (d) est un petit ARN interférent (siRNA) qui par interaction avec les ARNm correspondants audit polynucléotide, conduira à leur dégradation.

Selon encore un autre mode de réalisation avantageux de l'utilisation telle que définie ci-dessus, ladite protéine telle que définie en (g) est un variant de la séquence SEQ ID NO:2 présentant une mutation associée au diabète ou à l'hyperinsulinisme.

Selon encore un autre mode de réalisation avantageux de l'utilisation telle que définie ci-dessus, ledit fragment tel que défini en (f) présente une séquence choisie parmi les séquences SEQ ID NO:7, SEQ ID NO:8, SEQ ID NO:9 et SEQ ID NO:10.

Les Inventeurs décrivent également un polynucléotide tel que défini ci-dessus, à l'exception :
- des fragments d'au moins 15 nucléotides consécutifs inclus dans les séquences présentant les numéros d'accès dans la base de données NCBI n° AX526723, n° AX526725 et n° AX526727,
- des EST présentant les numéros d'accès dans la base de données GenBank BM565129, BM310003, BM875526, BG655918, BQ417284, BQ267316, BU072134, BQ267526, BQ270198, BU581447, BU070173, BQ631692 et BU949895, ainsi que des séquences présentant les numéros d'accès dans la base de données NCBI AX526723, AX526725 et AX526727.

Les fragments tels que définis ci-dessus peuvent notamment être utilisés comme sondes ou comme amorces pour détecter/amplifier des polynucléotides (ARN ou ADN génomique) correspondants au polynucléotide tel que défini ci-dessus dans d'autres organismes.

Préférentiellement, les paires d'amorces utilisables sont celles correspondants aux paires définies par les séquences SEQ ID NO:3 et SEQ ID NO:4 et par les séquences SEQ ID NO:5 et SEQ ID NO:6.

Les Inventeurs décrivent également les polynucléotides susceptibles d'être obtenus par amplification à l'aide des amorces telles que définies ci-dessus.

Selon un mode de réalisation avantageux dudit polynucléotide, il s'agit d'un petit ARN interférent correspondant au polynucléotide tel que défini ci-dessus, d'une taille inférieure à 30 nucléotides, de préférence d'une taille comprise entre 20 et 23 nucléotides, qui par interaction avec les ARNm correspondants audit polynucléotide, conduira à leur dégradation. Ces siRNA peuvent être obtenus par toute méthode connue de l'Homme du Métier, par exemple par synthèse chimique ou encore par expression à partir d'un vecteur.

Les Inventeurs décrivent les oligonucléotides sens correspondants aux polynucléotides définis plus haut qui, par interaction avec des protéines impliquées dans la régulation de l'expression dudit polynucléotide, induiront soit une inhibition, soit une activation de cette expression.

Les sondes et amorces telles que définies ci-dessus peuvent être marquées directement ou indirectement par un composé radioactif ou non radioactif par des méthodes bien connues de l'Homme du Métier, afin d'obtenir un signal détectable et/ou quantifiable.

Le marquage des amorces ou des sondes telles que définies ci-dessus est réalisé par des éléments radioactifs ou par des molécules non radioactives. Parmi les isotopes radioactifs utilisés, on peut citer le ³²P, le ³³P, le ³⁵S, le ³H ou l'¹²⁵I. Les entités non radioactives sont sélectionnées parmi les ligands tels que la biotine, l'avidine, la streptavidine, la digoxygénine, les haptènes, les colorants, les agents luminescents tels que les agents radioluminescents, chémoluminescents, bioluminescents, fluorescents, phosphorescents.

Les polynucléotides telles que définis ci-dessus peuvent ainsi être utilisés comme amorce et/ou sonde dans des procédés mettant en oeuvre notamment la technique de PCR (amplification en chaîne par polymérase) (U.S. N° 4,683,202). D'autres techniques d'amplification de l'acide nucléique cible peuvent être avantageusement employées comme alternative à la PCR. Il existe actuellement de très nombreux procédés permettant cette amplification, comme par exemple la technique SDA (Strand Displacement Amplification) ou technique d'amplification à déplacement de brin, la technique TAS (Transcription-based Amplification System), la technique 3SR (Self-Sustained Sequence Replication), la technique NASBA (Nucleic Acid Sequence Based Amplification), la technique TMA (Transcription Mediated Amplification), la technique LCR (Ligase Chain Reaction), la technique de RCR (Repair Chain Reaction), la technique CPR (Cycling Probe Reaction), la technique d'amplification à la Q-bêta-réplicase. On peut encore citer la PCR-SSCP qui permet de détecter des mutations ponctuelles.

Ces techniques sont bien entendu parfaitement connues de l'Homme du Métier.

Comme sondes ou comme amorces, les différents polynucléotides définis plus haut permettent, soit de déterminer le profil de transcription du gène correspondant ou une éventuelle altération de ce profil dans un échantillon biologique, soit de mettre en évidence le gène correspondant, des variants alléliques de ce gène ou une éventuelle altération fonctionnelle de ce gène (changement substantiel dans l'activité de la protéine codée par ledit gène) résultant d'une mutation (insertion, délétion ou substitution) d'un ou plusieurs nucléotides au niveau d'au moins un exon dudit gène. De telles mutations incluent en particulier les délétions, les insertions ou les substitutions non-conservatives au niveau de codons correspondant à des résidus d'acides aminés situés dans un domaine essentiel pour l'activité biologique de la protéine.

Ainsi les Inventeurs décrivent une méthode de détermination du profil de transcription du gène correspondant au polynucléotide défini ci-dessus ou d'une altération dudit profil, dans un échantillon biologique, comprenant une première étape d'obtention des ARN totaux à partir de l'échantillon biologique, une deuxième étape de mise en contact desdits ARN avec une sonde marquée constituée d'un polynucléotide tel que défini plus haut dans des conditions appropriées d'hybridation entre les ARN et la sonde et une troisième étape de révélation par tout moyen approprié des hybrides formés.

Selon un mode de mise en oeuvre de ladite méthode, la deuxième étape peut être une étape de rétrotranscription et/ou d'amplification des transcrits réalisée à l'aide d'une paire d'amorces telle que décrite précédemment et la troisième étape, une étape de révélation par tout moyen approprié des acides nucléiques amplifiés formés.

Ladite méthode de détermination du profil de transcription du gène peut en outre comporter une étape d'évaluation du taux de transcription du gène par comparaison avec un échantillon témoin préalablement choisi et éventuellement l'étude de sa corrélation avec un phénotype détectable comme par exemple le taux de proinsuline convertie en insuline mature, le contenu des cellules en insuline, le taux d'insuline secrétée en réponse à une stimulation par le glucose, la concentration intracellulaire ou intravésiculaire en zinc ou encore la quantité de protéine (produit du gène) exprimée à la surface de la cellule). Ledit échantillon témoin peut être constitué par exemple par un échantillon biologique présentant une transcription normale ou altérée du gène correspondant au polynucléotide défini plus haut auquel ladite méthode de détermination du profil de transcription du gène est appliquée dans les mêmes conditions.

il est également décrit ici une méthode de mise en évidence du gène correspondant au polynucléotide défini ci-dessus ou des variants alléliques dudit gène ou d'une altération fonctionnelle de ce gène, dans un échantillon biologique, comprenant une première étape d'obtention par tout moyen approprié de l'ADN à partir de l'échantillon biologique, une deuxième étape de mise en contact desdits ADN avec une sonde marquée constituée d'un polynucléotide tel que défini plus haut dans des conditions appropriées d'hybridation spécifique entre les ADN et la sonde et une troisième étape de révélation par tout moyen approprié des hybrides formés.

Selon un mode de mise en oeuvre avantageux de ladite méthode la deuxième étape peut être une étape d'amplification réalisée à l'aide d'une paire d'amorces telle que décrite précédemment et la troisième étape, une étape de révélation par tout moyen approprié des acides nucléiques amplifiés formés. La méthode peut éventuellement comporter une quatrième étape d'isolement et de séquençage des acides nucléiques mis en évidence.

Cette dernière méthode peut également permettre d'isoler un allèle du gène correspondant au polynucléotide défini plus haut, associé à un phénotype détectable, comme par exemple une variation de la glycémie post-prandiale, la présence ou non d'une sécrétion d'insuline, le taux de glucose circulant ou d'insuline secrétée en réponse à une stimulation par du glucose, et d'une manière générale à une pathologie de type diabète de type I ou II ou encore à une anomalie du métabolisme du zinc. Le zinc excrété au cours de la libération de l'insuline agit sur les canaux potassiques, responsables par l'intermédiaire de canaux calciques de cette exocytose. Il y a donc une boucle de rétrocontrôle [14]. Le polypeptide défini ci-dessus est impliqué dans l'accumulation du zinc dans les vésicules contenant l'insuline, et se retrouve également au cours de l'exocytose sur la membrane plasmique. Des mutants de la protéine pourraient donc modifier soit la quantité de zinc présent dans les vésicules, soit la concentration péricellulaire en zinc, et donc modifier l'état d'ouverture du canal potassique, aboutissant suivant l'effet de la mutation à une diminution ou une augmentation de l'excrétion d'insuline (diabète de type I ou hyperinsulinisme). Dans cette méthode particulière, l'échantillon biologique sera un échantillon provenant d'un individu exprimant ledit phénotype détectable.

Ces méthodes, particulièrement celles basées sur la recherche de mutations dans le gène, peuvent permettre la mise en évidence de façon préventive d'une prédisposition au diabète, ou le diagnostic du diabète ou de toute maladie liée au diabète, ou l'adaptation en terme de molécule ou de posologie du ou des traitements antidiabétiques.
est également décrite ici une trousse de réactifs pour la mise en oeuvre des méthodes précédemment décrites comprenant :
a) au moins une sonde ou une paire d'amorces telles que décrites plus haut ;
b) les réactifs nécessaires à la mise en oeuvre d'une réaction d'hybridation entre ladite sonde et/ou lesdites amorces et l'acide nucléique de l'échantillon biologique à tester ;
c) les réactifs nécessaires à la mise en oeuvre d'une réaction d'amplification ;
d) les réactifs nécessaires à la détection et/ou au dosage de l'hybride formé entre ladite sonde et l'acide nucléique de l'échantillon biologique ou des acides nucléiques amplifiés formés.

Une telle trousse peut également contenir des contrôles positifs ou négatifs afin d'assurer la qualité des résultats obtenus. Elles peuvent également contenir les réactifs nécessaires à la préparation des acides nucléiques à partir de l'échantillon biologique.

Le polynucléotide défini ci-dessus peut être utilisé *in vitro,* comme moyen d'étude :
a) de la surexpression du transporteur (ZnT-8) dans des lignées de cellules modèles (insulinome de rat INS-1 par exemple) et de l'impact sur la sécrétion de l'insuline en réponse à une stimulation par le glucose ;
b) de la sensibilité des cellules à la mort cellulaire (apoptose) induite par des conditions de stress oxydant ou de concentration faible ou forte en zinc ;
c) des étapes de différenciation de cellules souches en cellules sécrétrices d'insuline en réponse à différentes stimulations exogène (facteurs de croissance, extraits pancréatiques).

Les Inventeurs décrivent également la protéine codée par le polynucléotide défini ci-dessus.

Par "protéine" on entenddésigner un enchaînement précis d'acides aminés, modifiés ou non, comportant ou non des acides aminés non naturels.

La protéine définie ci-dessus est obtenue soit à partir d'une cellule bêta, soit par synthèse chimique, soit par les techniques d'ADN recombinant, notamment à partir de vecteur d'expression comprenant un insert constitué par polynucléotide tel que défini ci-dessus.

La protéine défini ci-dessus, lorsqu'elle est obtenue par synthèse chimique, peut être obtenue par l'une des nombreuses voies de synthèses peptidiques connues, par exemple les techniques mettant en oeuvre des phases solides ou des techniques utilisant des phases solides partielles, par condensation de fragments ou par une synthèse en solution classique. Dans ce cas la séquence de la protéine peut être modifiée afin d'améliorer sa solubilité, en particulier dans les solvants aqueux. De telles modifications sont connues de l'Homme du Métier comme par exemple la délétion de domaines hydrophobes ou la substitution d'acides aminés hydrophobes par des acides aminés hydrophiles.

De préférence, une telle protéine est une protéine comprenant ou présentant la séquence SEQ ID NO:2 (correspondant à la protéine codée par le gène *ZnT-8*).

Les Inventeurs décrivent également un fragment de la protéine telle que définie ci-dessus, **caractérisé en ce qu**'il est sélectionné dans le groupe constitué par les séquences SEQ ID NO:7, SEQ ID NO:8, SEQ ID NO:9 et SEQ ID NO:10, ainsi qu'une puce à protéine comprenant une protéine ou un fragment de protéine tels que définis ci-dessus.

La protéine, le fragment de protéine ou la puce à protéine tels que définis ci-dessus peuvent être utilisées pour détecter la présence d'anticorps dirigés contre lesdites protéines, dans le sérum d'un individu.

Les Inventeurs décrivent également l'utilisation de la protéine ou du fragment de protéine tel que définis ci-dessus, pour des mesures par des méthodes immunochimiques et immunoenzymatiques, ainsi que la recherche d'auto-anticorps dirigés contre la protéine selon l'Invention.

Un vecteur de clonage et/ou d'expression dans lequel est inséré le polynucléotide défini ci-dessus est également décrit.

Un tel vecteur peut contenir les éléments nécessaires à l'expression et éventuellement à la sécrétion de la protéine dans une cellule hôte.

Lesdits vecteurs comportent de préférence : un promoteur, des signaux d'initiation et de terminaison de la traduction, ainsi que des régions appropriées de régulation de la transcription. Ils doivent pouvoir être maintenus de façon stable dans la cellule et peuvent éventuellement comprendre des séquences codant pour des signaux particuliers spécifiant la sécrétion de la protéine traduite tels que par exemple un promoteur fort de nature ubiquitaire, ou un promoteur sélectif d'un type de cellule et/ou de tissu particulier, comme par exemple le pancréas. Ces différentes séquences de contrôle sont choisies en fonction de l'hôte cellulaire utilisé.

Le polynucléotide défini ci-dessus peut être inséré dans des vecteurs à réplication autonome au sein de l'hôte choisi ou des vecteurs intégratifs de l'hôte choisi.

Parmi les systèmes à réplication autonome, on utilise de préférence en fonction de la cellule hôte, des systèmes de type plasmidique ou viral. Les vecteurs viraux peuvent notamment être des adénovirus, des rétrovirus, des lentivirus, des poxvirus ou des virus herpétiques. L'Homme du Métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral; de tels virus sont, par exemple, les rétrovirus, ou les virus associés aux adénovirus (Adeno-associated virus ou AAV).

Parmi les vecteurs non viraux, on préfère les polynucléotides nus tels que l'ADN ou l'ARN nu, les chromosomes artificiels de bactérie (BAC, bacterial artificiel chromosome), les chromosomes artificiels de levure (YAC, yeast artificiel chromosome) pour l'expression dans la levure, les chromosomes artificiels de souris (MAC, mouse artificial chromosome) pour l'expression dans les cellules murines et de manière préférée les chromosomes artificiels d'homme (HAC, human artificial chromosome) pour l'expression dans les cellules humaines.

De tels vecteurs sont préparés selon les méthodes couramment utilisées par l'Homme du Métier, et les vecteurs recombinants en résultant peuvent être introduits dans l'hôte approprié par des méthodes standards, telles que par exemple la lipofection, l'électroporation, le choc thermique, la transformation après perméabilisation chimique de la membrane, la fusion cellulaire.

La protéine définie ci-dessus peut être purifiée selon les techniques connues de l'Homme du Métier. Ainsi, la protéine peut être purifiée à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps monoclonaux ou polyclonaux spécifiques, etc.. De préférence, la protéine est purifiée selon une méthode comprenant une première étape de séparation des protéines membranaires par centrifugation suivie d'une deuxième étape de purification par immunoaffinité selon la méthode décrite par T.C. Thomas, M.G. McNamee, (Purification of membrane proteins. Section IX, pp 499-520, in Methods in Enzymology, Guide to Protein Purification, Edité par M.P. Deutscher, vol 182, Academic press, New-York, 1990").

Les Inventeurs ont également pu montrer, par une étude du potentiel transmembranaire, que la structure secondaire de la protéine de l'Invention présente 3 boucles d'acides aminés extracellulaires (acides aminés 96-106, 164-176, 241-245) contre lesquelles peuvent être produits des anticorps monoclonaux ou polyclonaux.

Sont également décrits des anticorps mono- ou polyclonaux, caractérisés en ce qu'ils sont capables de reconnaître spécifiquement une protéine telle que définie ci-dessus.

De préférence, les anticorps reconnaissent spécifiquement la protéine de séquence SEQ ID NO:2, ses fragments ou les variants de ladite protéine tels que définis ci-dessus.

De manière préférée, ces anticorps reconnaissent spécifiquement les boucles extracellulaires de la protéine selon l'Invention correspondant aux SEQ ID NO:7, SEQ ID NO:8 et SEQ ID NO:10 (PEP1, PEP2 et PEP4) et/ou une boucle intracellulaire de la protéine selon l'Invention correspondant à la SEQ ID NO:9 (PEP3).

Ces anticorps sont, par exemple, des anticorps chimériques, des anticorps humanisés, des fragments Fab ou F(ab')2. Ils peuvent également se présenter sous forme d'immunoconjugués ou d'anticorps marqués afin d'obtenir un signal détectable et/ou quantifiable.

Lesdits anticorps peuvent être obtenus directement à partir de sérum humain, ou à partir de sérum d'animaux immunisés avec les protéines telles que définies ci-dessus, notamment produites par recombinaison génétique ou par synthèse peptidique.

Les anticorps polyclonaux spécifiques peuvent être obtenus selon les modes opératoires usuels. Les anticorps monoclonaux spécifiques peuvent être obtenus selon la méthode classique de culture d'hybridomes.

Une puce à protéine comprenant au moins un anticorps tel que défini ci-dessus est également décrite.

Les Inventeurs décrivent également l'utilisation des anticorps ou d'une puce à anticorps tels que définis ci-dessus, pour la détection et/ou la purification d'une protéine telle que définie plus haut, de préférence des boucles extracellulaires ou intracellulaires de ladite protéine, de manière préférée des séquences correspondant aux SEQ ID NO:7 à SEQ ID NO:10.

De manière générale, ces anticorps peuvent être avantageusement utilisés dans toute situation où l'expression d'une protéine telle que décrite ci-dessus, normale ou mutée, doit être observée.

Particulièrement, les anticorps monoclonaux, peuvent être utilisés pour la détection de ces protéines dans un échantillon biologique. Ils constituent ainsi un moyen d'analyse immunocytochimique ou immunohistochimique de l'expression des protéines définies ci-dessus, notamment la protéine de séquence SEQ ID NO:2 ou l'une de ses variantes, sur des coupes de tissus spécifiques. Généralement pour de telles analyses les anticorps utilisés sont marqués afin d'être détectables par exemple par des composés immunofluorescents, par marquage à l'or ou sous forme d'immunoconjugués enzymatiques.

Ils peuvent permettre notamment de mettre en évidence une expression anormale de ces protéines dans les tissus ou prélèvements biologiques.

Est également décrite une méthode de détection dans un échantillon biologique de la protéine ZnT-8, comprenant une première étape de mise en contact de l'échantillon biologique avec un anticorps tel que défini ci-dessus et une deuxième étape de mise en évidence par tout moyen approprié du complexe antigène-anticorps formé.

Une trousse permettant de mettre en oeuvre le procédé ci-dessus décrit comprend :
a) au moins un anticorps monoclonal ou polyclonal tel que défini plus haut ;
b) les réactifs permettant la détection du complexe antigène-anticorps produit lors de la réaction immunologique.

Selon un mode de réalisation particulier, la trousse peut éventuellement comprendre des réactifs pour la constitution d'un milieu permettant la réaction immunologique.

La présente Invention porte sur l'utilisation d'un anticorps reconnaissant spécifiquement un épitope sélectionné dans le groupe constitué par les peptides de séquence SEQ ID Nos: 7, 8, 9 et 10, pour la détection et/ou le tri des îlots de Langerhans, préférentiellement des cellules bêta, à partir d'un échantillon de pancréas humain ou animal, notamment le pancréas de souris, rat, lapin et de porc. Ce tri peut être réalisé par un appareillage de cytométrie en flux (FACS) pour ce qui concerne les cellules isolées. Pour les îlots, leur marquage pourrait améliorer les méthodes de séparation actuelle : séparation par gradient de densité avec du Ficoll, de l'Euro-Ficoll ou du Ficoll-Diatrizoate de sodium ou la méthode de choix qui est un gradient d'albumine sur un séparateur de cellules.

Ainsi, l'Invention a pour objet une méthode de sélection des cellules bêta des îlots de Langerhans, comprenant une première étape de mise en contact des cellules d'un échantillon biologique susceptible de contenir de tels îlots et/ou cellules avec un anticorps reconnaissant spécifiquement un épitope sélectionné dans le groupe constitué par les peptides de séquence SEQ ID Nos: 7, 8, 9 et 10" une deuxième étape de mise en évidence par tout moyen approprié des cellules marquées par l'anticorps et une troisième étape d'isolement par tout moyen approprié des cellules marquées.

Les anticorps reconnaissant spécifiquement un épitope sélectionné dans le groupe constitué par les peptides de séquence SEQ ID Nos: 7, 8, 9 et 10 peuvent également être utilisés, selon l'Invention, pour suivre le processus de différenciation de cellules souches en cellules bêta des îlots de Langerhans, particulièrement humaines ou animales, ainsi que pour trier ces cellules exprimant la protéine ZnT-8, particulièrement la protéine de séquence SEQ ID NO:2 après différenciation.

Ainsi, l'Invention a pour objet une méthode pour suivre le processus de différenciation de cellules souches en cellules d'îlot pancréatique ou en cellules bêta, comprenant une étape de mise en contact des cellules d'un échantillon biologique susceptible de contenir lesdites cellules souches en cours de différenciation avec un anticorps reconnaissant spécifiquement un épitope sélectionné dans le groupe constitué par les peptides de séquence SEQ ID Nos: 7, 8, 9 et 10, une deuxième étape de mise en évidence par tout moyen approprié des cellules marquées par l'anticorps et une troisième étape de visualisation par tout moyen approprié des cellules marquées.

Ladite méthode peut en outre comprendre une étape supplémentaire d'isolement par tout moyen approprié des cellules marquées.

Selon une mise en oeuvre préférée des utilisations et méthodes de l'Invention, l'anticorps reconnaissant spécifiquement un épitope sélectionné dans le groupe constitué par les peptides de séquence SEQ ID Nos: 7, 8, 9 et 10 est un anticorps chimérique, un anticorps humanisé, ou un fragment Fab ou F(ab')2. Cet anticorps peut également être marqué, et/ou compris dans une puce à protéine.

Outre les dispositions qui précèdent, l'Invention comprend encore d'autres dispositions qui ressortiront de la description qui va suivre, qui se réfère à des exemples de mise en oeuvre de l'Invention ainsi qu'aux dessins annexés, dans lesquels :

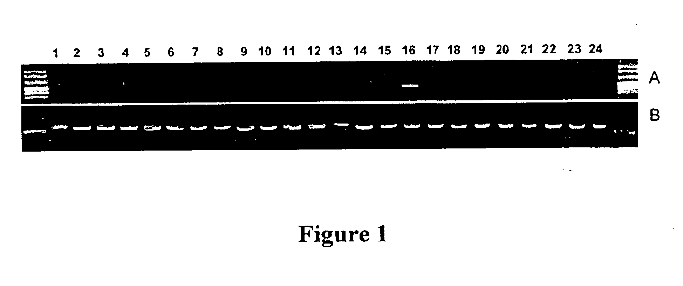

- la figure 1 illustre l'analyse par RT-PCR de l'expression tissulaire de l'ARN messager codant pour la protéine ZnT-8 (A), par comparaison avec l'expression du messager ubiquitaire de l'actine (B). 1 : Cerveau, 2 : Coeur, 3 : Rein, 4 : Rate, 5 : Foie, 6 : Colon, 7 : Poumon, 8 : intestin grêle, 9 : Muscle, 10 : Estomac, 11 : Testicule, 12 : Placenta, 13 : Glandes salivaires, 14 : Thyroïde, 15 :Glandes surrénales, 16 : Pancréas, 17 : Ovaires, 18 : Utérus, 19 : Prostate, 20 : Peau, 21 : Leucocytes, 22 : Moelle osseuse, 23 : Cerveau foetal, 24 : Foie foetal. L'expression dudit ARNm est détecté uniquement dans le pancréas (piste 16).

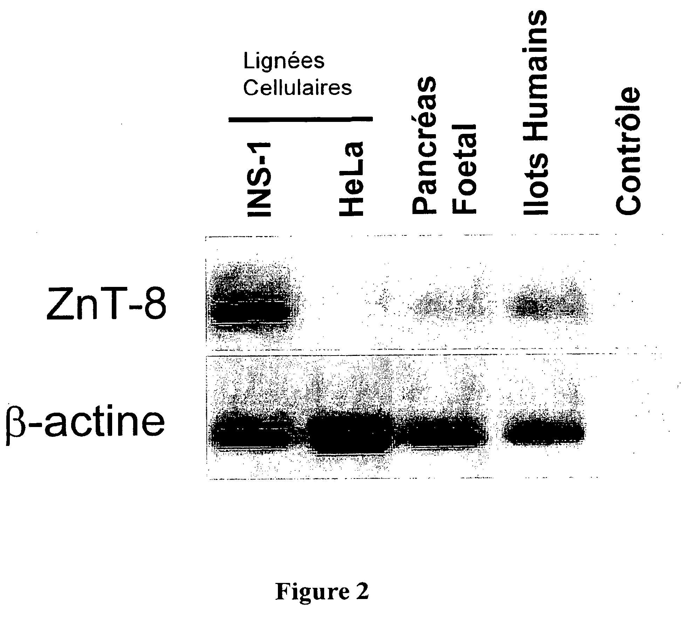

- la figure 2 illustre l'analyse par RT-PCR de l'expression de l'ARN messager codant pour la protéine ZnT-8, dans : une lignée d'insulinome de rat (INS-1, piste 1), des îlots pancréatiques humains foetaux (piste 2) et adultes (piste 3), par comparaison avec une lignée de cellules épithéliales (lignée Hela). L'ARNm est détecté dans la lignée d'insulinome de rat et les îlots pancréatiques adultes et foetaux, alors qu'aucun transcrit n'est détecté dans les cellules épithéliales (lignée Hela). Le messager de l'actine est utilisé comme contrôle.

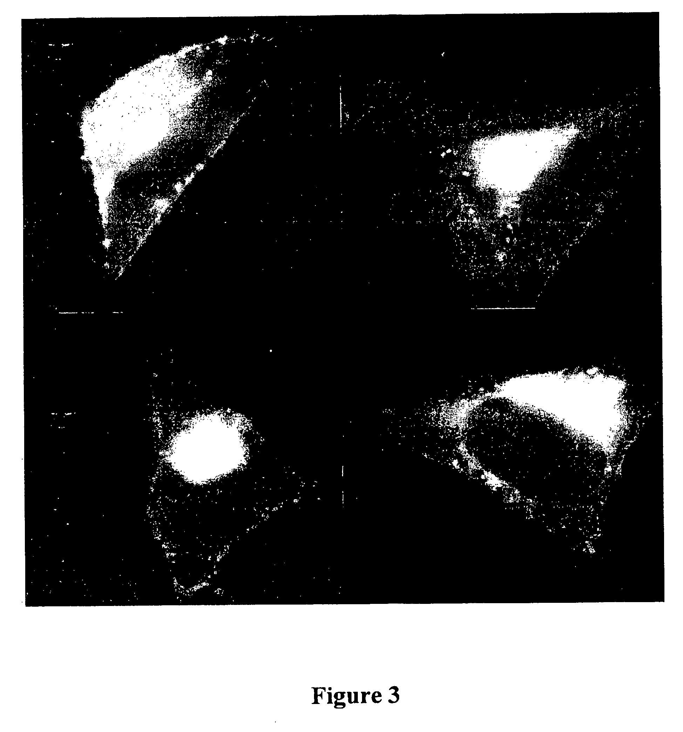

- la figure 3 illustre l'analyse en microscopie à fluorescence, de la localisation de la protéine de fusion ZnT-8-GFP dans des cellules épithéliales (lignée hela) transfectées. La fluorescence est localisée dans les vésicules intracytoplamiques, ainsi qu'au niveau de la membrane plasmique.

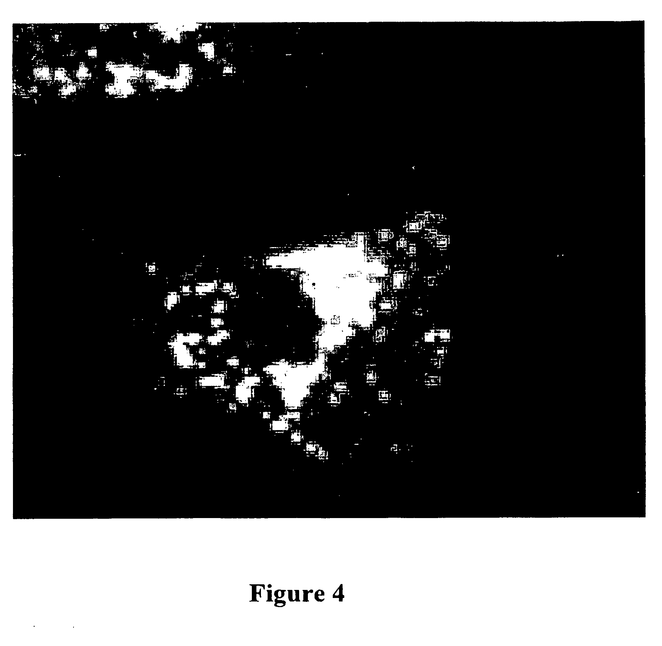

- la figure 4 illustre l'analyse en microscopie à fluorescence, de la localisation de la protéine de fusion ZnT-8-GFP dans des cellules d'insulinome de rat (lignée INS-1) transfectées. La fluorescence est localisée dans les vésicules de sécrétion indiquant un rôle de Znt-8 dans la maturation et l'exocytose de l'insuline.

Les exemples suivants sont illustratifs de l'Invention et ne la limitent aucunement.

### EXEMPLE 1 : Clonage de l'ADNc codant pour la protéine Znt-8

Le gène codant pour la protéine Znt-8, dénommé gène *Znt-8*, a été identifié par bioinformatique, par recherche de gènes homologues à ceux de la famille *ZnT,* en utilisant les séquences du génome humain disponibles. L'analyse des séquences génomiques a permis de localiser et de définir l'organisation intron/exon du gène *Znt-8.*

L'ADNc codant pour ZnT-8 a été amplifiée par RT-PCR à partir d'ARNm d'îlots de pancréas humains préparés selon la technique donnée dans Kenmochi T. et al. (Pancreas, 2000, 20, 2, 184-90), à l'aide de la paire d'amorces (SEQ ID NO:5 : 5'-ACTCTAGAATGGAGTTTCTTGAAAGAACGT A et SEQ ID NO:6 : 5'-AATCTA GAGTCACAGGGGTCTTCACAGA.), définies à partir de la séquence du gène *Znt-8.*

De manière plus précise, les ARN totaux ont été extraits à partir des îlots, en utilisant le kit "RNA extraction kit" (Roche) selon les instructions du fabricant. Les ARN ainsi obtenus ont été dosés par mesure de l'absorbance à 250 nm et conservés à -80°C.

L'amplification a été réalisée en utilisant le kit "Titan one tube RT-PCR kit" (Roche) selon les instruction du fabriquant, et la paire d'amorces SEQ ID NO:5 et SEQ ID NO:6. La transcription inverse a été réalisée à 52°C pendant 30 min puis les ADNc synthétisés ont été amplifies par 30 cycles (30 s à 94°C, 30 s à 53°C, 1 min à 72°C) et une élongation finale de 5 min à 72°C. Les produits d'amplification ont été séparés par électrophorèse en gel d'agarose (1,5 %) en présence de bromure d'éthydium et le produit d'amplification de 1123 paires de bases comprenant la séquence d'ADNc présentant la séquence SEQ ID NO:1 a été purifié à l'aide du kit Nucleic acid purification kit (QIAGEN), en suivant les instructions du fabricant.

### EXEMPLE 2 : Analyse de l'expression tissulaire de l'ARN messager codant pour la protéine Znt-8

L'expression de l'ARN messager codant pour la protéine Znt-8 a été analysée par PCR sur des ADNc commerciaux préparés à partir de différents tissus humains en utilisant les amorces suivantes : SEQ ID NO:3 : 5'-GAT GCT GCC CAC CTC TTA ATT GAC et SEQ ID NO:4 : 5'-TCA TCT TTT CCA TCC TGG TCT TGG. Les amorces (SEQ ID NO:3 et SEQ ID NO:4) ont été choisies dans 2 exons différents pour éviter l'amplification d'une séquence génomique. Les tissus testés sont : 1 : Cerveau, 2 : Coeur, 3 : Rein, 4 : Rate, 5 : Foie, 6 : Colon, 7 : Poumon, 8 : intestin grêle, 9 : Muscle, 10 : Estomac, 11 : Testicule, 12 : Placenta, 13 : Glande salivaire, 14 : Thyroïde, 15 : Corticosurrénale, 16 : Pancréas, 17 : Ovaire, 18 : Utérus, 19 : Prostate, 20 : Peau, 21 : Leucocytes sanguins, 22 : Moelle osseuse, 23 : Cerveau foetal, 24 : Foie foetal.

De manière plus précise : 2 µl d'ADNc ont été mélangés avec les 2 amorces spécifiques (1µM final) et un mélange classique de PCR (1 unité de Taq DNA polymerase, tampon avec 1,5 mM magnésium, dNTP 10 mM). L'amplification a été réalisée par 30 cycles (30 s à 94°C, 30 s à 53°C, 1 min à 72°C) et une étape finale d'élongation de 5 min à 72°C. Les produits ont ensuite été analysés par électrophorèse en gel d'agarose (1,5 %) en présence de bromure d'éthydium.

Les résultats illustrés par la figure 1 montrent que par comparaison avec le messager de l'actine utilisé comme contrôle (figure 1B), l'ARNm correspondant au polynucléotide de l'Invention est exprimé uniquement dans les cellules de pancréas (piste 16 de la figure 1A) mais pas dans les cellules des 23 autres tissus analysés (Pistes 1 à 15 et 17 à 24 de la figure 1A).

### EXEMPLE 3 : Expression de l'ARN messager correspondant au polynucléotide de l'Invention dans des cellules de pancréas foetal et adulte et dans une lignée d'insulinome de rat (INS-1)

L'analyse de l'expression de l'ARN messager codant pour la protéine ZnT-8 a été réalisée par RT-PCR, à partir d'ARN de différents tissus humains : îlots pancréatiques humains adultes et foetaux, lignée d'insulinome de rat (INS-1 ; Asfari M. et al., Endocrinology, 1992, 130, 1, 167-78), par comparaison avec une lignée de cellules épithéliales humaines (Hela), utilisée comme contrôle. 10⁶ cellules sont lavées deux fois avec du tampon phosphate (PBS) puis centrifugées 3 min à 2000 g. Les ARN totaux sont extraits comme décrit à l'exemple 1 et la concentration en ARN est ajustée à 1 ng/µl pour ZnT-8 ou 1 pg/µl pour le contrôle β-actine. Les transcrits sont amplifiés puis les produits d'amplification sont analysées, comme décrit à l'exemple 2.

Les résultats illustrés par la figure 2, montrent que l'ARNm codant pour la protéine ZnT-8 est exprimé dans les cellules des îlots pancréatiques humains foetaux et adultes et dans une lignée d'insulinome de rat (INS-1) mais pas dans une lignée de cellules épithéliales (lignée Hela).

### EXEMPLE 4 : Expression d'une protéine de fusion ZnT-8/GFP

La protéine est la protéine humaine, codée par l'ADNc correspondant à la séquence SEQ ID NO:1 (ZnT-8). Le produit PCR de 1123 paires de bases obtenu selon l'exemple 1 a été digéré avec l'enzyme de restriction *XbaI*, puis cloné dans le vecteur pcDNA3.1-CT-GFP (Invitrogen), pour donner le vecteur dénommé pZnT-8-GFP qui a été vérifié par séquençage.

Le vecteur pZnT-8-GFP a été transfecté de façon transitoire dans une lignée de cellules épithéliales (Hela) et de façon stable dans une lignée d'insulinome de rat (INS-1).

Les cellules épithéliales HeLa (ATCC numéro CCL-2) sont cultivées dans le milieu Opti-MEM (Modified Eagle's Medium, Life Technologies) supplémenté avec 5 % de sérum de veau décomplémenté et 2 mM de glutamine. Les cellules sont incubées à 37°C dans une atmosphère humidifiée enrichie à 5 % en CO₂.

Les cellules INS-1 sont cultivées dans du milieu RPMI (Life Technologies) supplémenté avec : sérum de veau foetal (10 %), 2-Mercaptoéthan-1-ol (50 µM), pyruvate de sodium (1 mM), HEPES (10 mM), L-glutamine (2 mM), penicilline 100 U/mL et streptomycine (100 µg/ml).

Les cellules cultivées dans des boites de Pétri de 35 mm sont transfectées avec le vecteur pZnT-8-GFP (1 µg d'ADN pour 10⁶ cellules), en utilisant le réactif Exgen500 (Euromedex) selon les instructions du fabricant. Après transfection par le vecteur ZnT-8-GFP, les cellules INS-1 sont sélectionnées, clonées dans le même milieu que précédemment additionné de 400 µg/mL de G418, puis la fluorescence des clones est observée sous un microscope à fluorescence inversé (Axiovert, Zeiss) en utilisant les paramètres suivants : longueur d'onde d'excitation : 450-490 nm ; longueur d'onde d'émission : 520 nm.

L'expression de la protéine de fusion ZnT-8-GFP dans les cellules Hela est analysée 48 heures après la transfection, par observation de la fluorescence comme précisé ci-dessus pour les clones de cellules INS-1 transfectées de façon stable.

Les résultats illustrés par la figure 3 (lignée Hela) montrent que la protéine ZnT-8 est localisée dans des vésicules intracytoplamiques, ainsi que sur la membrane plasmique ; cette localisation démontre que la protéine ZnT-8 emprunte la voie d'excrétion extracellulaire et se retrouve à la surface de la cellule.

Les résultats illustrés par la figure 4 (lignée INS-1) montrent que la protéine ZnT-8 est localisée dans les vésicules de sécrétion de l'insuline ; cette localisation indique que ZnT-8 est impliquée dans la maturation de l'insuline ; en outre, dans la mesure où cette expérience est réalisée à l'état basal (en l'absence de stimulation par le glucose) la protéine sera également présente sur la membrane plasmique au cours de l'exocytose de l'insuline, après stimulation par le glucose.

### EXEMPLE 5 : Analyse de la séquence de la protéine ZnT-8

L'analyse de la séquence primaire de ZnT-8 et la prédiction des domaines transmembranaires ont été réalisées avec les programmes TMpred (*http* ://www.*ch.embnet.org*/*software*/*TMPRED_form. htm*) et SOSUI (*http :*//*sosui.proteome.bio.tuat.ac.jp*/*sosuimenu0.html*).

La protéine ZnT-8 complète possède 6 domaines transmembranaires prédits (acides aminés 74-95, 107-126, 141-163, 177-196, 216-240, 246-267), les extrémités N- et C-terminales étant situées dans le cytoplasme.

### EXEMPLE 6 : Préparation d'un anticorps polyclonal dirigé contre les boucles extracellulaires (PEP1, PEP2 et PEP4) et contre une boucle intracellulaire (PEP3) de ZnT-8

Les peptides correspondant aux épitopes présentant les séquences SEQ ID NO:7 : PEP1 : HIAGSLAVVTDAAHLL ; SEQ ID NO:8 : PEP2 : CERLLYPDYQIQATV ; SEQ ID NO:9 : PEP3 CLGHNHKEVQANASVR, SEQ ID NO:10 : PEP4 : YFKPEYKIADPIC ont été synthétisés en phase solide, selon la méthode originellement décrite par Merrifield et al. (*J. Am*. *Chem.* Soc., 1964, **85**: 2149-) (1964), purifiés et conjugués à une protéine porteuse (albumine, par exemple). Les peptides conjugués sont injecté à des lapins selon le protocole d'immunisation suivant :

J0 : première immunisation ; J14 : seconde immunisation ; J28 : Troisième immunisation ; J38 : vérification de la spécificité ; J56 : quatrième immunisation ; J66 et J80 : récupération du sérum. Le sérum, peut être utilisé directement ou après purification sur colonne de protéine A avec une élution en milieu acide. Ces opérations ont été effectuées à façon par la société Eurogentec SA, Belgique.

### EXEMPLE 7 : Marquage fluorescent de l'anticorps obtenu à l'exemple 6

L'anticorps est purifié par chromatographie d'affinité sur une colonne de protéine G (Pharmacia). La colonne (1ml), équilibrée avec un tampon phosphate de sodium 10 mM, NaCl 0,15 M, pH 7,4 est chargée avec 5 ml de sérum, puis lavée avec 20 ml du même tampon pour éluer les protéines non fixées. L'anticorps est ensuite décroché avec une solution de glycine, HCl 0,1M, pH 2,5, puis neutralisé par 40µl de tampon Tris HCl 2M, pH 10,0.

2 mg d'anticorps sont dilués dans 1 ml de tampon phosphate pH 8.0. Une solution de NHS-FITC (SIGMA ; 1 mg/ml dans DMSO) est préparée extemporanément. 75 µl de la solution NHS-FITC est mélangé avec la solution d'anticorps puis le mélange est incubé à température ambiante pendant 45 minutes. L'anticorps marqué est purifié sur colonne PD-10 (PHARMACIA) de la façon suivante : la colonne est lavée avec 30ml de PBS, chargée avec 1ml de la solution d'anticorps marqué à purifier, puis avec 5 ml de PBS et des fractions de 2 ml sont ensuite collectés ; la seconde fraction contenant l'anticorps marqué est conservée.

### RÉFÉRENCES BIBLIOGRAPHIQUES

1. Soria B. : In-vitro differentiation of pancreatic beta-cells. Differentiation 2001 ; 68 : 205-19.
2. Gores PF, Sutherland DE. Pancreatic islet transplantation : is purification necessary ? Am. J. Surg. 1993 ; 166 : 538-42.
3. Bloc A, Cens T, Cruz H, Dunant Y. Zinc-induced changes in ionic currents of clonal rat pancreatic-cells : activation of ATP-sensitive K+ channels. J. Physiol. 2000 ; 529 Pt 3 : 723-34.
4. Meyer K, Irminger JC, Moss LG, de Vargas LM, Oberholzer J, Bosco D, et al. : Sorting human beta-cells consequent to targeted expression of green fluorescent protein. Diabetes 1998 ; 47 : 1974-7.
5. Giordano C, Stassi G, Todaro M, Richiusa P, Giordano M, Mattina A, et al. : Autofluorescence-activated sorting of human single beta cells and endocrine non-beta cells after enzymatic islet dissociation. Transplant Proc ; 1994 ; 26 : 651-2.
6. Lukowiak B, Vandewalle B, Riachy R, Kerr-Conte J, Gmyr V, Belaich S, et al. : Identification and purification of functional human beta-cells by a new specific zinc fluorescent probe. J. Histochem. Cytochern. 2001 ; 49 : 519-28.
7. Shiroi A, Yoshikawa M., Yokota H., Fukui H., Ishizaka S., Tatsumi K, et al. : Identification of Insulin-Producing Cells Derived from Embryonic Stem Cells by Zinc-Chelating Dithizone. Stem Cells 2002 ; 20 : 284-292.
8. Jiao L, Gray DW, Gohde W, Flynn GJ, Morris PJ. In vitro staining of islets of Langerhans for fluorescence-activated cell sorting. Transplantation 1991 ; 52 : 450-2.
9. Kallan AA, Roep BO, Arden SD, Hutton JC, de Vries RR. : Beta-cell reactive T-cell clones from type I diabetes patients are not beta cell specific and recognize multiple antigens. J. Autoimmun. 1995 ; 8 : 887-99.
10. Zalewski PD, Millard SH, Forbes IJ, Kapaniris 0, Slavotinek A, Betts WH et al. : Video image analysis of labile zinc in viable pancreatic islet cells using a specific fluorescent probe for zinc. J. Histochem. Cytochem. 1994 ; 42 : 877-84.
11. Easom RA. Beta-granule transport and exocytosis. Semin. Cell. Dev. Biol. 2000 ; 11 : 253-66.
12. Palmiter RD, Findley SD. Cloning and functional characterization of a mammalian zinc transporter that confers résistance to zinc. Embo J. 1995 ; 14 : 639-49.
13. Sambrook J, Russell DW. (2000) Molecular Cloning. A Laboratory Manual, Cold Spring Harbor Laboratory Press.
14. Bloc A et al, J Physiol. 2000, Dec. 15 ; 529 Pt 3 : 723-34)
15. Tomita T. Pathology International 2002 ; 52 : 425-432.

## Revendications

1. Utilisation d'un anticorps reconnaissant spécifiquement un épitope sélectionné dans le groupe constitué par les peptides de séquence SEQ ID Nos: 7, 8, 9 et 10, pour la détection et/ou le tri des îlots de Langerhans et/ou de cellules béta des îlots de Langerhans, à partir d'un échantillon de pancréas humain ou animal.

2. Utilisation selon la revendication 1, **caractérisée en ce que** ledit anticorps est un anticorps chimérique, un anticorps humanisé, ou un fragment Fab ou F(ab')2.

3. Utilisation selon la revendication 1 ou la revendication 2, **caractérisée en ce que** ledit anticorps est marqué.

4. Utilisation selon l'une quelconque des revendications 1 à 3, **caractérisée en ce que** ledit anticorps est compris dans une puce à protéine.

5. Utilisation selon l'une quelconque des revendications 1 à 4, dans laquelle l'échantillon de pancréas animal provient d'un animal sélectionné parmi les souris, rats, lapins et les porcs.

6. Méthode de sélection des cellules béta des îlots de Langerhans, comprenant une première étape de mise en contact des cellules d'un échantillon biologique susceptible de contenir de tels îlots et/ou cellules avec un anticorps reconnaissant spécifiquement un épitope sélectionné dans le groupe constitué par les peptides de séquence SEQ ID Nos: 7, 8, 9 et 10, une deuxième étape de mise en évidence des cellules marquées par l'anticorps et une troisième étape d'isolement des cellules marquées.

7. Utilisation d'un anticorps reconnaissant spécifiquement un épitope sélectionné dans le groupe constitué par les peptides de séquence SEQ ID Nos: 7, 8, 9 et 10, pour suivre le processus de différenciation de cellules souches en cellules béta des îlots de Langerhans.

8. Méthode pour suivre le processus de différenciation de cellules souches en cellules d'îlot pancréatique ou en cellules béta, comprenant une étape de mise en contact des cellules d'un échantillon biologique susceptible de contenir lesdites cellules souches en cours de différenciation avec un anticorps reconnaissant spécifiquement un épitope sélectionné dans le groupe constitué par les peptides de séquence SEQ ID Nos: 7, 8, 9 et 10, une deuxième étape de mise en évidence des cellules marquées par l'anticorps et une troisième étape de visualisation des cellules marquées.

9. Utilisation selon la revendication 7, ou méthode selon la revendication 6 ou la revendication 8, **caractérisée en ce que** ledit anticorps est un anticorps chimérique, un anticorps humanisé, ou un fragment Fab ou F(ab')2.

10. Utilisation ou méthode selon l'une quelconque des revendications 6 à 9, **caractérisée en ce que** ledit anticorps est marqué.

11. Utilisation ou méthode selon l'une quelconque des revendications 6 à 10, **caractérisée en ce que** ledit anticorps est compris dans une puce à protéine.

## Patentansprüche

1. Verwendung eines Antikörpers, der spezifisch ein Epitop erkennt, das ausgewählt ist aus der Gruppe bestehend aus den Peptiden der Sequenzen SEQ ID NO: 7, 8, 9 und 10, zur Erkennung und/oder Sortierung von Langerhans-Inseln und/oder β-Zellen der Langerhans-Inseln in einer humanen oder tierischen Pankreasprobe.

2. Verwendung nach Anspruch 1, **dadurch gekennzeichnet, dass** der Antikörper ein chimärer Antikörper, ein humanisierter Antikörper oder ein Fab- oder F(ab')2-Fragment ist.

3. Verwendung nach Anspruch 1 oder Anspruch 2, **dadurch gekennzeichnet, dass** der Antikörper markiert ist.

4. Verwendung nach einem der Ansprüche 1 bis 3, **dadurch gekennzeichnet, dass** der Antikörper in einem Proteinchip enthalten ist.

5. Verwendung nach einem der Ansprüche 1 bis 4, wobei die tierische Pankreasprobe von einem Tier stammt, das ausgewählt ist aus Mäusen, Ratten, Kaninchen und Schweinen.

6. Verfahren zur Selektion von β-Zellen der Langerhans-Inseln, umfassend einen ersten Schritt des In-Kontakt-Bringens der Zellen einer biologischen Probe, die solche Inseln und/oder Zellen enthalten kann, mit einem Antikörper, der spezifisch ein Epitop erkennt, das ausgewählt ist aus der Gruppe bestehend aus den Peptiden der Sequenzen SEQ ID NO: 7, 8, 9 und 10, einen zweiten Schritt des Nachweises der mit dem Antikörper markierten Zellen, und einen dritten Schritt des Isolierens der markierten Zellen.

7. Verwendung eines Antikörpers, der spezifisch ein Epitop erkennt, das ausgewählt ist aus der Gruppe bestehend aus den Peptiden der Sequenzen SEQ ID NO: 7, 8, 9 und 10, zum Verfolgen des Differenzierungsprozesses von Stammzellen zu β-Zellen der Langerhans-Inseln.

8. Verfahren zum Verfolgen des Differenzierungsprozesses von Stammzellen zu pankreatischen Inselzellen oder β-Zellen, umfassend einen Schritt des In-Kontakt-Bringens der Zellen einer biologischen Probe, die die Stammzellen, die differenzieren, enthalten kann, mit einem Antikörper, der spezifisch ein Epitop erkennt, das ausgewählt ist aus der Gruppe bestehend aus den Peptiden der Sequenzen SEQ ID NO: 7, 8, 9 und 10, eine zweiten Schritt des Nachweises der mit dem Antikörper markierten Zellen, und einen dritten Schritt des Sichtbarmachens der markierten Zellen.

9. Verwendung nach Anspruch 7 oder Verfahren nach Anspruch 6 oder Anspruch 8, **dadurch gekennzeichnet, dass** der Antikörper ein chimärer Antikörper, ein humanisierter Antikörper oder ein Fab- oder F(ab')2-Fragment ist.

10. Verwendung oder Verfahren nach einem der Ansprüche 6 bis 9, **dadurch gekennzeichnet, dass** der Antikörper markiert ist.

11. Verwendung oder Verfahren nach einem der Ansprüche 6 bis 10, **dadurch gekennzeichnet, dass** der Antikörper in einem Proteinchip enthalten ist.

## Claims

1. Use of an antibody that specifically recognises an epitope selected from the group consisting of the peptides of sequence SEQ ID Nos: 7, 8, 9 and 10 for detecting and/or sorting islets of Langerhans and/or beta cells of the islets of Langerhans, starting from a sample of human or animal pancreas.

2. Use according to claim 1, **characterised in that** said antibody is a chimeric antibody, a humanised antibody or a Fab or F(ab')2 fragment.

3. Use according to claim 1 or claim 2, **characterised in that** said antibody is labelled.

4. Use according to any one of claims 1 to 3, **characterised in that** said antibody is contained in a protein chip.

5. Use according to any one of claims 1 to 4, wherein the sample of animal pancreas is from an animal selected from mice, rats, rabbits and pigs.

6. Method for selecting beta cells of the islets of Langerhans, comprising a first step of bringing the cells of a biological sample that may contain such islets and/or cells into contact with an antibody that specifically recognises an epitope selected from the group consisting of the peptides of sequence SEQ ID Nos: 7, 8, 9 and 10, a second step of detecting the cells labelled with the antibody, and a third step of isolating the labelled cells.

7. Use of an antibody that specifically recognises an epitope selected from the group consisting of the peptides of sequence SEQ ID Nos: 7, 8, 9 and 10 for monitoring the process of differentiation of stem cells into beta cells of the islets of Langerhans.

8. Method for monitoring the process of differentiation of stem cells into pancreatic islet cells or into beta cells, comprising a step of bringing the cells of a biological sample that may contain said stem cells in the process of differentiating into contact with an antibody that specifically recognises an epitope selected from the group consisting of the peptides of sequence SEQ 10 Nos: 7, 8, 9 and 10, a second step of detecting the cells labelled with the antibody, and a third step of visualising the labelled cells.

9. Use according to claim 7, or method according to claim 6 or claim 8, **characterised in that** said antibody is a chimeric antibody, a humanised antibody or a Fab or F(ab')2 fragment.

10. Use or method according to any one of claims 6 to 9, **characterised in that** said antibody is labelled.

11. Use or method according to any one of claims 6 to 10, **characterised in that** said antibody is contained in a protein chip.


### SEQUENCE LISTING

<110> COMMISSARIAT A L'ENERGIE ATOMIQUE
   UNIVERSITE JOSEPH FOURIER
   SEVE Michel
   FAVIER Alain
<120> Protéine spécifique des cellules pancréatiques bêta des îlots de
   Langerhans et ses applications.
<130> CGA263S85
<160> 10
<170> PatentIn version 3.1
<210> 1
   <211> 1110
   <212> DNA
   <213> Homo sapiens
<400> 1
   atggagtttc ttgaaagaac gtatcttgtg aatgataaag ctgccaagat gcatgctttc     60
   acactagaaa gtgtggaact ccaacagaaa ccggtgaata aagatcagtg tcccagagag    120
   agaccagagg agctggagtc aggaggcatg taccactgcc acagtggctc caagcccaca    180
   gaaaaggggg cgaatgagta cgcctatgcc aagtggaaac tctgttctgc ttcagcaata    240
   tgcttcattt tcatgattgc agaggtcgtg ggtgggcaca ttgctgggag tcttgctgtt    300
   gtcacagatg ctgcccacct cttaattgac ctgaccagtt tcctgctcag tctcttctcc    360
   ctgtggctgt catcgaagcc tccctctaag cggctgacat ttggatggca ccgagcagag    420
   atccttggtg ccctgctctc catcctgtgc atctgggtgg tgactggcgt gctagtgtac    480
   ctggcatgtg agcgcctgct gtatcctgat taccagatcc aggcgactgt gatgatcatc    540
   gtttccagct gcgcagtggc ggccaacatt gtactaactg tggttttgca ccagagatgc    600
   cttggccaca atcacaagga agtacaagcc aatgccagcg tcagagctgc ttttgtgcat    660
   gcccttggag atctatttca gagtatcagt gtgctaatta gtgcacttat tatctacttt    720
   aagccagagt ataaaatagc cgacccaatc tgcacattca tcttttccat cctggtcttg    780
   gccagcacca tcactatctt aaaggacttc tccatcttac tcatggaagg tgtgccaaag    840
   agcctgaatt acagtggtgt gaaagagctt attttagcag tcgacggggt gctgtctgtg    900
   cacagcctgc acatctggtc tctaacaatg aatcaagtaa ttctctcagc tcatgttgct    960
   acagcagcca gccgggacag ccaagtggtt cggagagaaa ttgctaaagc ccttagcaaa   1020
   agctttacga tgcactcact caccattcag atggaatctc cagttgacca ggaccccgac   1080
   tgccttttct gtgaagaccc ctgtgactag                                    1110
<210> 2
   <211> 369
   <212> PRT
   <213> Homo sapiens
<400> 2
   Met Glu Phe Leu Glu Arg Thr Tyr Leu Val Asn Asp Lys Ala Ala Lys
   1               5                   10                  15
   Met His Ala Phe Thr Leu Glu Ser Val Glu Leu Gln Gln Lys Pro Val
               20                  25                  30
   Asn Lys Asp Gln Cys Pro Arg Glu Arg Pro Glu Glu Leu Glu Ser Gly
           35                  40                  45
   Gly Met Tyr His Cys His Ser Gly Ser Lys Pro Thr Glu Lys Gly Ala
       50                  55                  60
   Asn Glu Tyr Ala Tyr Ala Lys Trp Lys Leu Cys Ser Ala Ser Ala Ile
   65                  70                  75                  80
   Cys Phe Ile Phe Met Ile Ala Glu Val Val Gly Gly His Ile Ala Gly
                   85                  90                  95
   Ser Leu Ala Val Val Thr Asp Ala Ala His Leu Leu Ile Asp Leu Thr
               100                 105                 110
   Ser Phe Leu Leu Ser Leu Phe Ser Leu Trp Leu Ser Ser Lys Pro Pro
           115                 120                 125
   Ser Lys Arg Leu Thr Phe Gly Trp His Arg Ala Glu Ile Leu Gly Ala
       130                 135                 140
   Leu Leu Ser Ile Leu Cys Ile Trp Val Val Thr Gly Val Leu Val Tyr
   145                 150                 155                 160
   Leu Ala Cys Glu Arg Leu Leu Tyr Pro Asp Tyr Gln Ile Gln Ala Thr
                   165                 170                 175
   Val Met Ile Ile Val Ser Ser Cys Ala Val Ala Ala Asn Ile Val Leu
               180                 185                 190
   Thr Val Val Leu His Gln Arg Cys Leu Gly His Asn His Lys Glu Val
           195                 200                 205
   Gln Ala Asn Ala Ser Val Arg Ala Ala Phe Val His Ala Leu Gly Asp
       210                 215                 220
   Leu Phe Gln Ser Ile Ser Val Leu Ile Ser Ala Leu Ile Ile Tyr Phe
   225                 230                 235                 240
   Lys Pro Glu Tyr Lys Ile Ala Asp Pro Ile Cys Thr Phe Ile Phe Ser
                   245                     250                     255
   Ile Leu Val Leu Ala Ser Thr Ile Thr Ile Leu Lys Asp Phe Ser Ile
               260                     265                     270
   Leu Leu Met Glu Gly Val Pro Lys Ser Leu Asn Tyr Ser Gly Val Lys
           275                     280                     285
   Glu Leu Ile Leu Ala Val Asp Gly Val Leu Ser Val His Ser Leu His
       290                     295                     300
   Ile Trp Ser Leu Thr Met Asn Gln Val Ile Leu Ser Ala His Val Ala
   305                     310                     315                     320
   Thr Ala Ala Ser Arg Asp Ser Gln Val Val Arg Arg Glu Ile Ala Lys
                   325                     330                     335
   Ala Leu Ser Lys Ser Phe Thr Met His Ser Leu Thr Ile Gln Met Glu
               340                     345                     350
   Ser Pro Val Asp Gln Asp Pro Asp Cys Leu Phe Cys Glu Asp Pro Cys
           355                     360                     365
   Asp
<210> 3
   <211> 24
   <212> DNA
   <213> Artificial sequence
<220>
   <223> Amorce
<400> 3
   gatgctgccc acctcttaat tgac 24
<210> 4
   <211> 24
   <212> DNA
   <213> Artificial sequence
<220>
   <223> Amorce
<400> 4
   tcatcttttc catcctggtc ttgg 24
<210> 5
   <211> 31
   <212> DNA
   <213> Artificial sequence
<220>
   <223> Amorce
<400> 5
   actctagaat ggagtttctt gaaagaacgt a 31
<210> 6
   <211> 28
   <212> DNA
   <213> Artificial sequence
<220>
   <223> Amorce
<400> 6
   aatctagagt cacaggggtc ttcacaga 28
<210> 7
   <211> 16
   <212> PRT
   <213> Homo sapiens
<400> 7
   His Ile Ala Gly Ser Leu Ala Val Val Thr Asp Ala Ala His Leu Leu
   1 5 10 15
<210> 8
   <211> 15
   <212> PRT
   <213> Homo sapiens
<400> 8
   Cys Glu Arg Leu Leu Tyr Pro Asp Tyr Gln Ile Gln Ala Thr Val
   1 5 10 15
<210> 9
   <211> 16
   <212> PRT
   <213> Homo sapiens
<400> 9
   Cys Leu Gly His Asn His Lys Glu Val Gln Ala Asn Ala Ser Val Arg
   1 5 10 15
<210> 10
   <211> 13
   <212> PRT
   <213> Homo sapiens
<400> 10
   Tyr Phe Lys Pro Glu Tyr Lys Ile Ala Asp Pro Ile Cys
   1 5 10